Europäisches Patentamt

European Patent Office

Office européen des brevets

(11) Publication number: **0 416 931 A2**

# EUROPEAN PATENT APPLICATION

(21) Application number: 90309797.0

(22) Date of filing: 07.09.90

(51) Int. Cl.⁵: **A61N 5/06**

(30) Priority: 07.09.89 GB 8920230
13.02.90 GB 9003165

(43) Date of publication of application:
**13.03.91 Bulletin 91/11**

(84) Designated Contracting States:
**DE FR GB IT**

(71) Applicant: **MEDICAL LASER TECHNOLOGIES
LIMITED
Research Park, Riccarton
Edinburgh EH14 4AP(GB)**

(72) Inventor: **Henderson, Brian
50 Brisbane Road
Largs, Ayrshire(GB)**
Inventor: **Colles, Michael John
36 Hope Terrace
Edinburgh EH9 9AR(GB)**

(74) Representative: **Pattullo, Norman et al
Ian G. Murgitroyd and Company Mitchell
House 333 Bath Street
Glasgow G2 4ER Scotland(GB)**

(54) **Medical applications for an optical device.**

(57) An optical device (4) is disclosed which comprises an optical fibre (3) and an isotropic optical radiator emitter/detector (2) located at one end of the fibre (3). The emitter/detector (2) may be inserted into animal tissue (5) in order to irradiate the tissue (5) adjacent to the emitter/detector (2) with optical radiation (71) which is coupled into the optical fibre and emitted by the emitter/detector (2). The device (4) may also be used to detect the fluorescence of a drug present in the tissue (5) in order to determine the activity of the drug. Other applications of the device include measuring optical parameters of animal tissue, for example the optical radiation transmission profile of the tissue.

FIG. 1

EP 0 416 931 A2

## MEDICAL APPLICATIONS OF AN OPTICAL DEVICE

This invention relates to an optical device and, in particular, to an optical device used in hyperthermic treatment and optical diagnosis of tissue.

The cells of animal tissue die if exposed to excessive heat, and this fact can be used to good effect in medical endoscopy by introducing an optical fibre to an area to be treated and passing infra red light along the fibre to irradiate the area. However, to be of greatest use the light should be emitted isotropically, that is with equal energy density in all directions.

An isotropic emitter and a method of manufacture thereof are at present the subject matter of our co-pending European Application No 90302852.0. This device has a specially fashioned tip which forms a spherical isotropic emitter/detector at the end of the fibre.

The fact that normal and infected tissue will reflect light to different degree and the fact that arterial plaque is fluorescent may be used in the optical diagnosis of animal and human tissue.

According to a first aspect of the present invention use of an optical device for the treatment of animal tissue, comprising inserting the optical device into animal tissue and passing optical radiation along said device, the device emitting the optical radiation to irradiate the tissue.

The optical device is preferably substantially isotropic.

According to a second aspect of the present invention use of an optical device for determining the activity of a drug during use of the drug in the treatment of animal tissue, comprising inserting the optical device into the tissue, irradiating the tissue with optical radiation to cause the drug to fluoresce, and detecting the fluorescence produced and wherein the optical device emits the optical radiation which irradiates the tissue and/or detects the fluorescence produced.

Optical radiation from the range of visible (green) to near infra-red may be used.

Preferably, an infra-red wavelength for example 1.06 $\mu$m is used for treating tissue, as infra-red radiation provides effective and gradual penetration and does not burn the tissue.

However, if it is the fluorescent characteristics of a drug that are to be monitored, a different wavelength may be more useful. For example, to induce fluorescence, radiation of 632nm could be used.

The optical device of the second aspect of the present invention may be used either to induce or to detect fluorescence. It may also be adapted to perform both functions; for example by either including two fibres in one device, or using a single fibre with appropriate optical equipment to differentiate between incoming and outgoing light. If two fibres in one device are used, one fibre may be used to provide radiation and the other to detect radiation. The optical properties and constants of tissue may be examined by the use of such a device, for example, radiation can be provided at one wavelength along one of the two fibres and the reflected radiation can be examined at the same wavelength along the other.

If used in photodynamic therapy, the device can be used to monitor the efficacy of a drug by examining its fluorescent characteristics. The detected light travels from the probe end through a filter which removes radiation of the wavelength used to induce fluorescence, for example 632nm, allowing the fluorescence alone, for example 692nm, to be detected.

Preferably, the material used in making the probe end is selected for its low absorption of light in these ranges.

Preferably the curable material is a chemical resin that has a catalyst that induces curing at a wavelength with an absorption band centred, for example, at 467nm; that has a viscosity which enables it to be suspended in an organic liquid such as paraffin to produce a spherical probe end, and that is transparent when cured.

According to a third aspect of the present invention use of an optical device, which comprises an optical fibre and an isotropic optical radiation emitter/detector located at one end of the fibre, for measuring an optical parameter of animal tissue comprising embedding the emitter/detector in the tissue, supplying optical radiation through the fibre to the emitter/detector, the emitter/detector emitting the optical radiation and detecting the intensity of the radiation reflected from the tissue by means of the emitter/detector.

Preferably, coherent optical radiation is used such as optical radiation from a laser. Most preferably of a visible wavelength such as approximately 630nm from a Helium Neon (HeNe) laser.

Preferably, the isotropic emitter includes impurities of a material such as $TiO_2$ or $SiO_2$ which scatter the light in all directions equally.

According to a fourth aspect of the present invention a method of calibrating an optical device having an optical radiation emitter/detector, the method comprising submerging the emitter/detector in a calibration fluid, passing optical radiation into the emitter/detector and detecting the quantity of radiation reflected by the fluid to obtain a calibration reading.

Preferably the calibration fluid is water.

The corrected reflectance readings are given by taking reflectance readings in the method described above and subtracting the calibration reading for reflectance of clean water also determined as described above.

The device may be used for a number of purposes including measurement of the oxygenation of blood and symmetry of tissue.

The device may also be used to determine the depth profile of tissue, that is the radiation intensity measurement obtained Vs depth of tissue which may be used to determine the effect of treatment on the tissue and the dosage of radiation used in cancer treatment may be adjusted as necessary.

Preferably, the depth profile is determined by irradiating the tissue from above and embedding the fibre and detector in the tissue at various depths and detecting the radiation intensity at those depths.

The dosage may be increased by increasing illumination from above or by using a fibre and emitter embedded in the tissue for in situ illumination.

Preferably, one fibre is used to supply optical radiation to the emitter/detector and to return optical radiation for measurement.

The fibre may be coupled to a second fibre, outwith the tissue, which leads to a measurement detector.

Alternatively, a supply fibre and a separate detector fibre may be embedded in the emitter/detector.

According to a fifth aspect of the present invention there is provided a method of introducing an optical device comprising an optical fibre and an isotropic emitter/detector at one end of the optical fibre, into animal tissue the method comprising inserting a hypodermic needle into the tissue passing the emitter/detector and the fibre through the needle and into the tissue, and removing the needle from the tissue.

Preferably, the fibre is of the order of 50 $\mu$m in diameter.

According to a sixth aspect of the present invention there is provided an optical device comprising an optical fibre wherein the fibre has a number of spaced sequential markings along its length.

Typically, the markings are equally spaced.

Typically, the optical device comprises an isotropic emitter/detector and, preferably, the markings on the fibre are formed from spots of substantially the same radiation cured resin as used in the emitter/detector.

Preferably, the markings are spaced at 1cm intervals.

Most preferably, every 5th and/or 10th mark is coloured so as to make counting the markings easier.

According to a seventh aspect of the present invention there is provided an optical device comprising an optical fibre and an isotropic optical radiation emitter/detector at one end of the optical fibre and wherein a section of the optical fibre adjacent the emitter/detector is clad in a moulded material.

Preferably, the moulded material is a light cured resin such as methyl methacrylate.

Preferably, the cladding enables the transmission of light and does not include scattering impurities.

According to an eighth aspect of the present invention there is provided a mount for an optical fibre comprising a syringe filter, the fibre being passed through the syringe filter and positioned centrally in the syringe filter by a tapered plug having a central bore which receives the fibre when the plug is positioned in the syringe filter.

Preferably, the plug is formed from a plastics material.

Preferably, the syringe is threaded for attachment to external apparatus such as an optical detector or standard mount.

According to a ninth aspect of the present invention there is provided an optical fibre probe for detecting arterial plaque comprising an optical fibre and an isotropic optical radiation emitter located at one end of the fibre, a length of the fibre adjacent the isotropic emitter being clad in a radiation cured material which is optically transparent in the wavelength range covering the fluorescence from artery plaque and wherein the material forms a detector.

Typically, the material is moulded to form a cylindrical detector and the length of the cylindrical detector enables the detection of optical radiation over a longer length of the artery than would have been possible with the isotropic detector.

Preferably, the material from which the detector is formed includes scattering impurities such as $TiO_2$ and $SiO_2$.

Preferably, a plurality of fibres are embedded in the cylindrical detector, the fibres transmitting the detected optical radiation back to a monitor and thus providing a directional indication of plaque positioned on the artery walls.

According to a tenth aspect of the present invention a method of manufacturing an optical fibre probe comprising placing a plastic sheath over a section of an optical fibre to create an annular gap between the fibre and the sheath, inserting a radiation curable material into the annular gap, passing radiation down the fibre to cure the material, and removing the sheath.

Most preferably, the sheath is formed from a material with a low coefficient of friction such as P.T.F.E.

As the plaque is fluorescent the isotropic emitter may be used simply to excite fluorescence in the plaque, which may be detected by the cylindrical detector.

The term "optical radiation" is used to denote radiation in the ultraviolet and infra-red regions of the electromagnetic spectrum as well as the visible region.

Embodiments of the present invention will now be described, by way of example, with reference to the accompanying drawings in which:-

Fig. 1 illustrates a method of treating tissue according to the first aspect of the present invention;

Fig. 2 illustrates a first method of detecting the fluorescence of a drug according to the second aspect of the present invention;

Fig. 3 illustrates a second method of detecting the fluorescence of a drug according to the second aspect of the invention;

Fig. 4 shows a schematic diagram of an optical device being used in tissue diagnosis using a method in accordance with the third aspect of the present invention;

Fig. 5 illustrates an optical fibre device in accordance with the sixth aspect of the present invention;

Fig. 6 shows a schematic diagram of a clad fibre in accordance with the seventh aspect of the present invention;

Fig. 7 shows a fibre mount in accordance with the eighth aspect of the present invention; and,

Figs. 8A and 8B shows a schematic diagram of an isotropic emitter and cylindrical detector in use in arterial plaque detection in accordance with the ninth aspect of the present invention.

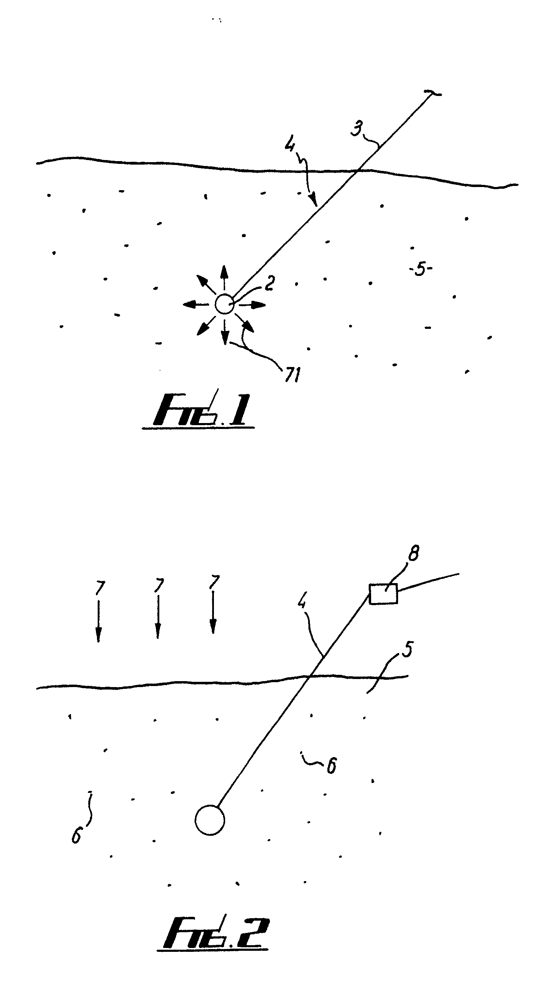

Fig. 1 shows an isotropic probe 4 which comprises a probe end 2 and an optical fibre 3. The probe 4 is located in tissue 5 and may be used to irradiate the tissue 5 adjacent to the probe end 2. Radiation of the desired wavelength, which is typically infra-red and may have a wavelength of 1.06 $\mu$m, is transmitted through the optical fibre 3 from a source laser (not shown) and the radiation is emitted isotropically by the probe end 2 into the surrounding tissue 5, as indicated by the arrows 71.

Because of the low resistance of living cells to excessive heat, the probe 4 may be used to eradicate tumours. It may do this with a precision lacking in present technology due to the shape of the probe end 2 and the ease with which the device can be placed internally in the body at the desired location, and so can be operated with the minimum of damage to healthy tissue.

The device may also be used for clearing arteries in angioplasty. The device, with its shaped probe end, may be fed into the artery and a laser may be used to fire light in predetermined patterns at the artery wall. A balloon device may then be used to push back the warmed artery wall.

These patterns are determined by the wavelength used and by the shape of the probe end, which may be in the form of a lens for distributing light as desired. Because different wavelengths react differently with the same shaped device, the pattern of light hitting the artery wall could be varied to suit the local circumstances as they change along the artery by suitable choice of wavelength used.

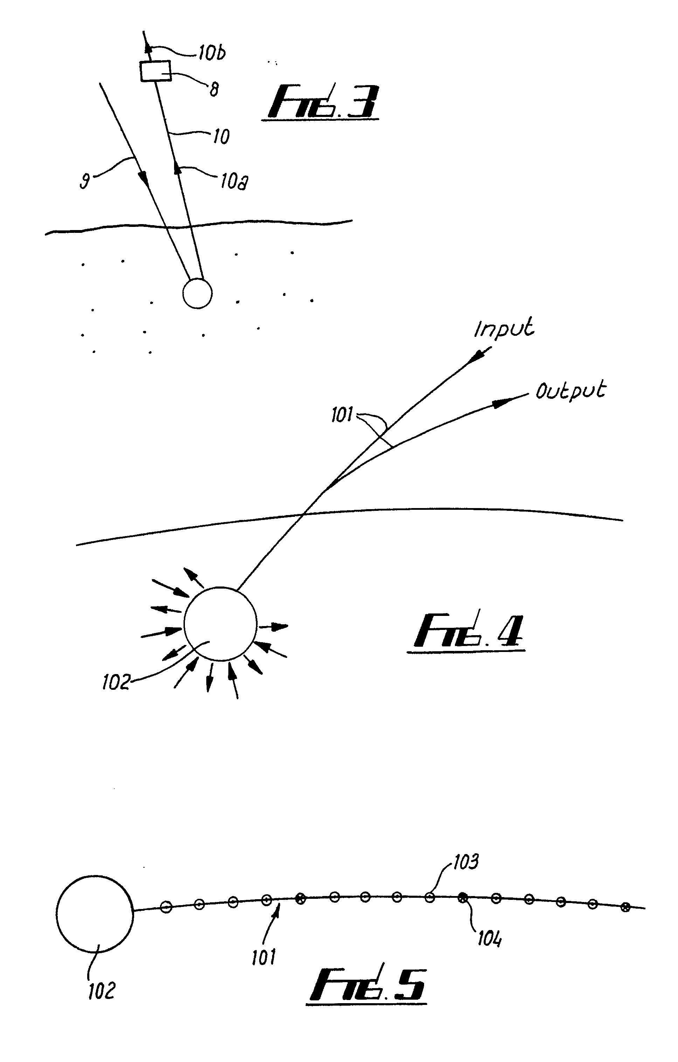

The activity of many drugs used in medical treatments can be monitored by examining changes in the drug's fluorescence at specific wavelengths (see Fig. 2). Tissue 5 is treated with a drug, which is absorbed as indicated at 6, and the tissue 5 is irradiated, as indicated at 7, at 488 nm to cause fluorescence of the drug. The level of fluorescence is then detected via an isotropic probe 4 which passes the detected light through a filter 8 which transmits the radiation produced by fluorescence but which does not transmit the irradiating radiation.

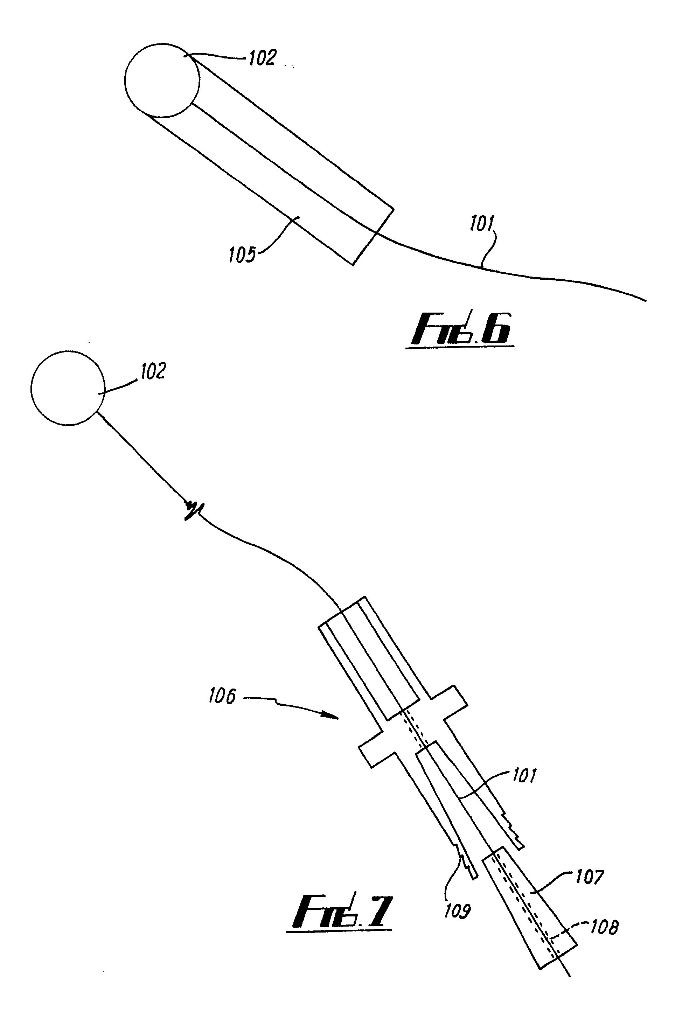

The probe 4 may also be used to irradiate the tissue 5 (see Fig. 3), or a separate probe may be used for each function. If it is the same probe, then it is preferable to use two optical fibres 3a, 3b, (Fig. 3) terminating within the sphere for emission 9 and detection 10 of the light respectively. Arrow 9 represents incoming light of irradiation and arrow 10 is light to be monitored after the light of irradiation and fluorescence 10a have been separated at 8. It is also possible to use a single fibre for both these functions.

This process can also be applied to detect the presence of viruses to which drugs are attached, and to obtain information about their content.

A further advantage of the device is that some embodiments of an isotropic probe end in accordance with the second aspect of the present invention may also be considered as an integrating sphere: all light inside the probe is evenly distributed and is the same at all internal points. Existing integrating spheres are large, and the present invention allows this effect to be created inside the body.

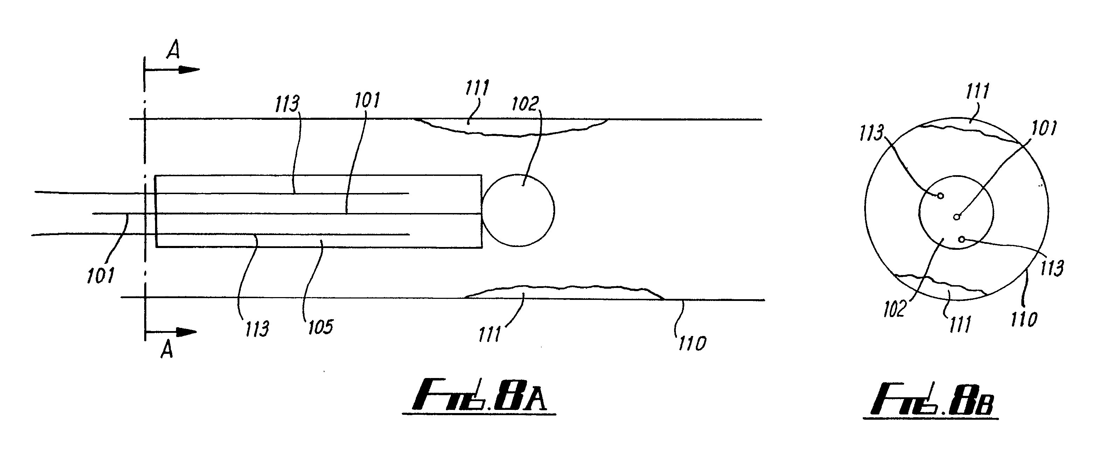

Fig. 4 shows a method of tissue optical parameter measurement wherein an optical fibre 101, one end of which comprises an isotropic light emitter/detector 102 is embedded in the tissue and light is supplied through the fibre 101 to the emitter/detector 102 which emits light into the tissue and detects light reflected from the tissue. A HeNe laser is used to supply the light required at a wavelength of approximately 630nm.

One fibre may be used to supply light to the emitter/detector and to return light for measurement or a second fibre 101 may be coupled to the first to return light for measurement or the second fibre 101 may be embedded in the emitter/detector for the same purpose.

Fig. 5 illustrates an optical fibre 101 comprising an emitter/detector 102 at one end of the fibre 101 and a plurality of equally spaced markings 103, in the form of spots of light cured material, along the fibre length. The markings 103 are spaced apart by approximately 1cm and each fifth mark 104 is coloured differently to the remaining marks 103 so as to ease countings of the marks 103 and 104.

Fig. 6 illustrates an embodiment of the optical device wherein a portion of fibre 101 adjacent the emitter/detector 102 is clad in a moulded material 5 to form a more robust and less easily damaged fibre. The moulded material is a light cured resin such as methyl methacrylate. The resin is not doped with scattering impurities.

Fig. 7 shows a fibre mount comprising a standard syringe filter 106, the fibre 101 being passed through the centre of the syringe filter 106 and positioned centrally in the syringe filter 106 by a tapered plug 107 with a narrow central bore 108 which receives the fibre 101, when the plug 107 is in position. The output end of the syringe filter 106 has a screw-thread 109 to enable the syringe filter 106 to be attached to an optical detector or standard optical mount, not shown.

This mount is of particular value when the optical device is inserted into the tissue by a method according to the fourth aspect of the present invention wherein a syringe needle is inserted into the tissue and the fibre 101 and emitter/detector 102 is passed through the syringe needle and into the tissue and needle is removed.

The syringe filter 106 constitutes a low cost disposable optical mount for use with the optical device.

Figs. 8A and 8B show an optical device being used in arterial plaque detection, the device comprising an optical fibre 101, one end of which includes an isotropic emitter 102, a cylindrical detector in the form of a length of fibre 101 adjacent the emitter 102 clad in a light cured moulded material 105. The moulded material 105 being transparent in the wavelength range covering both the fluorescence from arterial plaque and the initial input wavelength of an excitation laser.

The length of the cylindrical detector 105 enables the detection of light over a longer length of artery than would be possible with the isotropic detector.

The detector 105 is doped with scattering impurities to improve the percentage of the light which is transmitted along the fibre.

The isotropic emitter 102 emits light which is reflected by the artery wall 110 and arterial plaque 111 to a different degree. Thus detection of the quantity of reflected light will provide an indication of the quantity of arterial plaque. As arterial plaque 111 is fluorescent the isotropic emitter 102 may be used simply to excite fluorescence in the plaque 111 which may be detected by the cylindrical detector 105.

In order to provide a directional indication of plaque 111 position, a plurality of fibres 113 may be embedded in the cylindrical detector 105, the fibres 113 transmitting the detected light back to a monitor and thus providing a directional indication of plaque position 111 on the artery walls 110 due to the difference in quantity of light detected by the various fibres 113.

The cylindrical detector 105 is manufactured by placing the P.T.F.E. sheath over the fibre 101 adjacent the emitter 102 and inserting optically curable moulding material into the space between the sheath and the fibre 101. Light is then passed down the fibre to cure the material and the sheath is removed to disclose the detector 105.

In other examples of the invention fibres having different diameters may be used, for example 500 μm.

Modifications and improvements may be incorporated without departing from the scope of the invention.

## Claims

1 Use of an optical device for the treatment of animal tissue (5), comprising inserting the optical device (4) into animal tissue (5) and passing optical radiation along said device, the device emitting the optical radiation (71) to irradiate the tissue.

2 Use of an optical device according to Claim 1, wherein the optical device (4) emits the optical radiation (71) substantially isotropically.

3 Use of an optical device for determining the activity of a drug during use of the drug in the treatment of animal tissue (5), comprising inserting the optical device (4) into the tissue (5), irradiating the tissue (5) with optical radiation (7) to cause the drug to fluoresce, and detecting the fluorescence produced and wherein the optical device (4) emits the optical radiation (7) which irradiates the tissue and/or detects the fluorescence produced.

4 Use of an optical device, which comprises an optical fibre (101) and an isotropic optical radiation emitter/detector (102) located at one end of the fibre (101), for measuring an optical parameter of animal tissue comprising embedding the emitter/detector (102) in the tissue, supplying optical radiation through the fibre (101) to the emitter/detector (102), the emitter/detector (102) emitting the optical radiation and detecting the intensity of the radiation reflected from the tissue by means of the emitter/detector (102).

5 Use of an optical device, which comprises an optical fibre and an isotropic optical radiation detector located at one end of the fibre, for measuring optical radiation absorption versus depth of tissue, comprising irradiating the tissue with optical radiation from an optical radiation emitter and embedding the detector at various separations from the position of the emitter and detecting the intensity of the optical radiation at those separations.

6 A method of introducing an optical device comprising an optical fibre and an isotropic emitter/detector at one end of the optical fibre into animal tissue the method comprising inserting a hypodermic needle into the tissue, passing the emitter/detector and the fibre through the needle and into the tissue, and removing the needle from the tissue.

7 An optical device comprising an optical fibre (101) wherein the fibre (101) has a number of spaced sequential markings (103, 104) along its length.

8 An optical device comprising an optical fibre (101) and an isotropic optical radiation emitter detector (102) at one end of the optical fibre and wherein a section of the optical fibre (101) adjacent the emitter/detector (102) is clad in a moulded material (105).

9 A method of calibrating an optical device having an optical radiation emitter/detector, the method comprising submerging the emitter/detector in a calibration fluid, passing optical radiation into the emitter/detector and detecting the quantity of radiation reflected by the fluid to obtain a calibration reading.

10 A mount for an optical fibre (101) comprising a syringe filter (106), the fibre (101) being passed through the syringe filter (106) and positioned centrally in the syringe filter (106) by a tapered plug (107) having a central bore (108) which receives the fibre (101) when the plug (107) is positioned in the syringe filter (106).

11 An optical fibre probe for detecting arterial plaque (111) comprising an optical fibre (101) and an isotropic optical radiation emitter (102) located at one end of the fibre (101), a length of the fibre adjacent the isotropic emitter (102) being clad in a radiation cured material which is optically transparent in the wavelength range covering the fluorescence from artery plaque (111) and wherein the material forms a detector (105).

12 A method of manufacturing an optical fibre probe comprising placing a plastic sheath over a section of an optical fibre to create an annular gap between the fibre and the sheath, inserting a radiation curable material into the annular gap, passing radiation down the fibre to cure the material, and removing the sheath.

_FIG.1_

_FIG.2_

*Fig.3*

*Fig.4*

*Fig.5*

102

101

105

**Fig.6**

102

106

101

107

109

**Fig.7**

108

FIG. 8A

FIG. 8B

EP 0 416 931 A2